# EUROPEAN PATENT SPECIFICATION

(11) **EP 3 389 475 B1**
(45) Date of publication and mention of the grant of the patent: **15.07.2020**
(21) Application number: 16823438.3
(22) Date of filing: 19.12.2016
(51) Int. Cl.: A61B 5/00, A61B 5/046, A61B 5/0464, A61B 5/042, A61B 5/0456

(54) **AUTOMATIC MAPPING USING VELOCITY INFORMATION**
AUTOMATISCHES MAPPING ANHAND VON GESCHWINDIGKEITSINFORMATIONEN
CARTOGRAPHIE AUTOMATIQUE UTILISANT DES INFORMATIONS SUR LA VITESSE

(30) Priority: 20.12.2015 US 201562270030 P
(43) Date of publication of application: 24.10.2018
(73) Proprietor: Boston Scientific Scimed Inc., Maple Grove, Minnesota 55311 (US)
(72) Inventor: HULTZ, Paul, Brookline, New Hampshire 03033 (US); PERLMAN, Mordechai, Cambridge, Massachusetts 02138 (US); BENNETT, Nathan H., Cambridge, Massachusetts 02138 (US); STEWART, Brian, North Reading, Massachusetts 01864 (US)
(74) Representative: Pfenning, Meinig & Partner mbB
(86) International application number: PCT/US2016/067536
(87) International publication number: WO 2017/112590

(56) References cited:
- EP-A1- 2 901 953
- WO-A1-2012/092016
- US-A1- 2012 184 863
- US-A1- 2015 216 438
- US-B1- 8 577 450
- US-B1- 8 812 091

## Description

### TECHNICAL FIELD

Embodiments of this disclosure relate to systems and methods for providing information about a patient's heart and, in particular, to systems and methods for electro-anatomically mapping a patient's heart.

### BACKGROUND

Use of minimally invasive procedures, such as catheter ablation, to treat a variety of heart conditions, such as supraventricular and ventricular arrhythmias, is becoming increasingly prevalent. Often, these procedures involve the mapping of electrical activity in the heart at various locations on the endocardial or epicardial surface, referred to as cardiac mapping, to identify the mechanism of the arrhythmia followed by a targeted ablation of the site. To perform the cardiac mapping, a catheter with one or more electrodes can be inserted into the patient's heart.

Cardiac mapping techniques include contact mapping, near contact mapping, and non-contact mapping. In contact mapping, one or more catheters are advanced into the heart and physiological signals resulting from the electrical activity of the heart are acquired with one or more electrodes located at the catheter distal tip after determining that the tip is in stable and steady contact with the endocardial surface of a heart chamber. The location and electrical activity can be measured on a point-by-point basis at, for example, about 50 to 200 points on the internal surface of the heart to construct an electro-anatomical depiction of the heart.

In near-contact mapping, a movable catheter having multiple spatially distributed electrodes is placed in a heart chamber of interest and moved to one or more locations within the chamber of interest, where the electrodes are on or near, such as within millimeters of, the endocardial surface of the heart chamber. Measurements are taken automatically at each of the locations of the catheter, without determining whether the electrodes are in contact with the surface of the heart. These measurements are analyzed to detect the endocardial surface of the heart chamber in the vicinity of the catheter. The location of the catheter, e.g., a location provided by a tracking system, and the measurements from the electrodes are used to reconstruct the chamber anatomy, where, for example, 20,000 measurements may be made to construct an electro-anatomical depiction of the heart. As the tracked catheter is moved inside the chamber, a partial or complete representation of the chamber anatomy can be constructed.

In non-contact mapping, a multiple electrode catheter is placed in the heart chamber of interest and the catheter is deployed to assume a three-dimensional shape. Using the signals detected by the non-contact electrodes and information on chamber anatomy and relative electrode location, the system calculates and provides physiological information regarding the endocardial surface of the heart chamber. In any of these cardiac mapping techniques, the generated map may then serve as the basis for deciding on a therapeutic course of action, such as tissue ablation, to alter the propagation of the heart's electrical activity and to restore normal heart rhythm.

For example, document US 2012/184863 A1 describes a method for providing information about a patient's heart. The method includes measuring signals from one or more electrodes at multiple positions in the heart cavity in response to electrical activity in the patient's heart cavity over multiple heart beat cycles, generating, by a computer, annotation information for the measured signals by applying one or more operators to the measured signals to identify at least one of regions of the heart having double deflections, regions of the heart having multiple deflections, regions of the heart having fractionation, regions of the heart having double activation, and regions of the heart having no activation. Moreover, the method includes generating, by the computer, an electroanatomical representation of the patient's heart that includes at least some of the annotation information.

As a further example, document WO 2012/092016 A1 describes a system for diagnosing arrhythmias and directing catheter therapies. The system may allow for measuring, classifying, analyzing, and mapping spatial electrophysiological (EP) patterns within a body. The system may further guide arrhythmia therapy and update maps as treatment is delivered. The system may use a medical device having a high density of sensors with a known spatial configuration for collecting EP data and positioning data. Further, the system may also use an electronic control system (ECU) for computing and providing the user with a variety of metrics, derivative metrics, high definition (HD) maps, HD composite maps, and general visual aids for association with a geometrical anatomical model shown on a display device

### SUMMARY

As stated above, before treating an arrhythmia the arrhythmia needs to be identified, which typically involves the mapping of electrical activity in the heart at various locations on the endocardial or epicardial surface. Many arrhythmias are caused by reentry of an activation signal, in which the activation signal propagates repeatedly around a fixed circuit. Reentry of an activation signal can occur when an activation signal includes a pathway where the activation signal propagates slower than usual. Due to the slow propagation of the activation signal, the cardiac tissue may have sufficient time to recover between cycles. Oftentimes, a pathway where the activation signal propagates slower than normal passes through a narrow strip of myocardium. As such, limited ablation along the pathway will often terminate a reentrant arrhythmia and, therefore, can be used as an effective treatment strategy. Throughout this disclosure, the term "slow pathway" may be used, interchangeably, to refer to a pathway where an activation signal propagates slower than the typical propagation speed for the particular patient under a particular set of circumstances (e.g., when the patient is at rest or in motion, when the patient is stressed or relaxed, when other physiological parameters have certain levels or characteristics, and/or the like).

Since reentries are oftentimes caused by slow pathways, it may be advantageous for a mapping system to facilitate the display of these slow pathways, in order to focus a user's attention. Accordingly, embodiments disclosed herein provide systems and methods for facilitating the display of slow pathways. The object of the present invention is achieved with the system according to claim 1 and with the method according to claim 8. Preferred embodiments are defined in the dependent claims. Exemplary disclosures include the following.
In Example 1, a system for facilitating display of cardiac mapping information comprises a mapping probe configured to sense a plurality of cardiac electrical signals; and a processing unit configured to: receive the plurality of cardiac electrical signals; determine a plurality of signal features, each of the plurality of signal features comprising a feature of one of the plurality of cardiac electrical signals; determine a plurality of conduction characteristics, each of the plurality of conduction characteristics based on one or more of the plurality of signal features; and generate, based on the plurality of signal features and the corresponding plurality of conduction characteristics, a cardiac map, the cardiac map comprising an anatomical shell and a set of annotations representing at least a portion of the plurality of signal features.
In Example 2, the system of Example 1, wherein the processing unit is further configured to determine a set of conduction characteristics, of the plurality of conduction characteristics, that satisfy a condition, wherein the set of annotations comprises a representation of each of the plurality of signal features that corresponds to one of the set of conduction characteristics that satisfy the condition.
In Example 3, a system for facilitating display of cardiac mapping information comprises a mapping probe configured to sense a plurality of cardiac electrical signals; and a processing unit configured to: receive the plurality of cardiac electrical signals; determine a plurality of signal features, each of the plurality of signal features comprising a feature of one of the plurality of cardiac electrical signals; determine a plurality of conduction characteristics, each of the plurality of conduction characteristics based on one or more of the plurality of signal features; determine a set of conduction characteristics, of the plurality of conduction characteristics, that satisfy a condition; and provide, for presentation on a display device, a cardiac map, the cardiac map comprising an anatomical shell and a representation of each of the plurality of signal features that corresponds to one of the set of conduction characteristics that satisfy the condition.
In Example 4, the system of Example 3, wherein the cardiac map does not include a representation of a signal feature that does not correspond to one of the set of conduction characteristics that satisfy the condition.
In Example 5, the system of any of Examples 3 and 4, wherein a conduction characteristic satisfies the condition when the conduction characteristic is within a particular range.
In Example 6, the system of any of Examples 3 and 4, wherein the conduction characteristics are conduction velocities and wherein a conduction characteristic satisfies the condition when the conduction characteristic is no greater than a threshold.
In Example 7, the system of any of Examples 3 and 4, wherein the conduction characteristics are activation gradients and wherein an activation gradient satisfies the condition when the activation gradient is no less than a threshold.
In Example 8, the system of any of Examples 3-7, wherein each of the plurality of signal features comprises at least one of an activation time, a minimum voltage value, a maximum voltage value, a maximum negative time-derivative of voltage, an instantaneous potential, a voltage amplitude, a dominant frequency, a mean cycle length, and a peak-to-peak voltage.
In Example 9, the system of Example 8, each of the plurality of signal features comprising an activation time, and wherein the processing unit is configured to determine the plurality of conduction characteristics by: determining a gradient associated with each of the plurality of activation times; and determining the inverse of the gradient associated with each of the plurality of activation times.
In Example 10, the system of Example 9, wherein each of the plurality of signal features comprises an activation time associated with a single beat.
In Example 11, the system of Example 8, wherein the processing unit is configured to determine the plurality of conduction characteristics by performing a least-squares computation using at least three electrode activation times, wherein each of the at least three electrode activation times is associated with one of at least three non-collinear electrode locations.
In Example 12, a method for facilitating display of cardiac mapping information comprises receiving, from a mapping probe, a plurality of cardiac electrical signals; determining a plurality of signal features, each of the plurality of signal features comprising a feature of one of the plurality of cardiac electrical signals; determining a plurality of conduction characteristics, each of the plurality of conduction characteristics based on one or more of the plurality of signal features; and causing a display device to display, based on the plurality of conduction characteristics, a cardiac map, the displayed cardiac map comprising an anatomical shell and a representation of at least one of the plurality of signal features.
In Example 13, the method of Example 12, further comprising: generating an activation map, the activation map comprising the anatomical shell, a plurality of vertices and a representation of each of a plurality of activation times at a vertex of the plurality of vertices; determining activation time differences for each vertex, wherein each of the plurality of conduction characteristics is determined based on one or more of the activation time differences; determining a set of conduction characteristics, of the plurality of conduction characteristics, that satisfy a condition, wherein a conduction characteristic satisfies the condition when the conduction characteristic is within a particular range; and wherein the displayed cardiac map does not include a representation of a signal feature that does not correspond to one of the set of conduction characteristics that satisfy the condition.
In Example 14, the method of Example 13, wherein determining an activation time difference of a vertex includes performing a least-squares computation using at least three activation times associated with the vertex and at least two other map vertices of the activation map.
In Example 15, the method of Example 12, wherein determining the plurality of conduction characteristics includes: performing a least-squares computation using at least three electrode activation times, wherein each of the at least three electrode activation times is associated with one of at least three non-collinear electrode locations of the mapping probe.
In Example 16, a system for facilitating display of cardiac mapping information comprises a mapping probe configured to sense a plurality of cardiac electrical signals; and a processing unit configured to: receive the plurality of cardiac electrical signals; determine a plurality of signal features, each of the plurality of signal features comprising a feature of one of the plurality of cardiac electrical signals; determine a plurality of conduction characteristics, each of the plurality of conduction characteristics based on one or more of the plurality of signal features; and generate, based on the plurality of signal features and the corresponding plurality of conduction characteristics, a cardiac map, the cardiac map comprising an anatomical shell and a set of annotations representing at least a portion of the plurality of signal features.
In Example 17, the system of Example 16, wherein a first annotation of the set of annotations is associated with a first map point lying on the anatomical shell, and wherein the processing unit is configured to generate the first annotation by performing an interpolation based on a set of vectors, wherein each of the set of vectors is associated with an additional map point and comprises one of the plurality of electrical signal features and a corresponding one of the plurality of conduction characteristics.
In Example 18, the system of Example 16, wherein the processing unit is further configured to determine a set of conduction characteristics, of the plurality of conduction characteristics, that satisfy a condition, wherein the set of annotations comprises a representation of each of the plurality of signal features that corresponds to one of the set of conduction characteristics that satisfy the condition and wherein the cardiac map does not include a representation of a signal feature that does not correspond to one of the set of conduction characteristics that satisfy the condition.
In Example 19, the system of Example 18, wherein a conduction characteristic satisfies the condition when the conduction characteristic is within a particular range.
In Example 20, the system of Example 18, wherein the conduction characteristics are conduction velocities and wherein a conduction characteristic satisfies the condition when the conduction characteristic is no greater than a threshold.
In Example 21, the system of Example 18, wherein the conduction characteristics are activation gradients and wherein an activation gradient satisfies the condition when the activation gradient is no less than a threshold.
In Example 22, the system of Example 16, wherein each of the plurality of signal features comprises at least one of an activation time, a minimum voltage value, a maximum voltage value, a maximum negative time-derivative of voltage, an instantaneous potential, a voltage amplitude, a dominant frequency, a mean cycle length, and a peak-to-peak voltage.
In Example 23, a system for facilitating display of cardiac mapping information comprises a mapping probe configured to sense a plurality of cardiac electrical signals; and a processing unit configured to: receive the plurality of cardiac electrical signals; determine a plurality of signal features, each of the plurality of signal features comprising a feature of one of the plurality of cardiac electrical signals; determine a plurality of conduction characteristics, each of the plurality of conduction characteristics based on one or more of the plurality of signal features; determine a set of conduction characteristics, of the plurality of conduction characteristics, that satisfy a condition; and provide, for presentation on a display device, a cardiac map, the cardiac map comprising an anatomical shell and a representation of each of the plurality of signal features that corresponds to one of the set of conduction characteristics that satisfy the condition.
In Example 24, the system of Example 23, wherein the cardiac map does not include a representation of a signal feature that does not correspond to one of the set of conduction characteristics that satisfy the condition.
In Example 25, the system of Example 23, wherein a conduction characteristic satisfies the condition when the conduction characteristic is within a particular range.
In Example 26, the system of Example 23, wherein the conduction characteristics are conduction velocities and wherein a conduction characteristic satisfies the condition when the conduction characteristic is no greater than a threshold.
In Example 27, the system of Example 23, wherein the conduction characteristics are activation gradients and wherein an activation gradient satisfies the condition when the activation gradient is no less than a threshold.
In Example 28, the system of Example 23, wherein each of the plurality of signal features comprises at least one of an activation time, a minimum voltage value, a maximum voltage value, a maximum negative time-derivative of voltage, an instantaneous potential, a voltage amplitude, a dominant frequency, a mean cycle length, and a peak-to-peak voltage.
In Example 29, the system of Example 28, each of the plurality of signal features comprising an activation time, and wherein the processing unit is configured to determine the plurality of conduction characteristics by: determining a gradient associated with each of the plurality of activation times; and determining the inverse of the gradient associated with each of the plurality of activation times.
In Example 30, the system of Example 29, wherein each of the plurality of signal features comprises an activation time associated with a single beat.
In Example 31, the system of Example 28, wherein the processing unit is configured to determine the plurality of conduction characteristics by performing a least-squares computation using at least three electrode activation times, wherein each of the at least three electrode activation times is associated with one of at least three non-collinear electrode locations.
In Example 32, a method for facilitating display of cardiac mapping information comprises receiving, from a mapping probe, a plurality of cardiac electrical signals; determining a plurality of signal features, each of the plurality of signal features comprising a feature of one of the plurality of cardiac electrical signals; determining a plurality of conduction characteristics, each of the plurality of conduction characteristics based on one or more of the plurality of signal features; and causing a display device to display, based on the plurality of conduction characteristics, a cardiac map, the displayed cardiac map comprising an anatomical shell and a representation of at least one of the plurality of signal features.
In Example 33, the method of Example 32, further comprising: generating an activation map, the activation map comprising the anatomical shell, a plurality of vertices and a representation of each of a plurality of activation times at a vertices of the plurality of vertices; determining activation time differences for each vertex, wherein each of the plurality of conduction characteristics is determined based on one or more of the activation time differences; determining a set of conduction characteristics, of the plurality of conduction characteristics, that satisfy a condition, wherein a conduction characteristic satisfies the condition when the conduction characteristic is within a particular range; and wherein the displayed cardiac map does not include a representation of a signal feature that does not correspond to one of the set of conduction characteristics that satisfy the condition.
In Example 34, the method of Example 32, wherein determining an activation time difference of a vertex includes performing a least-squares computation using at least three activation times associated with the vertex and at least two other map vertices of the activation map.
In Example 35, the method of Example 32, wherein determining the plurality of conduction characteristics includes: performing a least-squares computation using at least three electrode activation times, wherein each of the at least three electrode activation times is associated with one of at least three non-collinear electrode locations of the mapping probe.

While multiple embodiments are disclosed, still other embodiments of the disclosed subject matter will become apparent to those skilled in the art from the following detailed description, which shows and describes illustrative embodiments of the disclosure. Accordingly, the drawings and detailed description are to be regarded as illustrative in nature and not restrictive.

### BRIEF DESCRIPTION OF THE DRAWINGS

FIG. 1A is a diagram illustrating an electro-anatomical mapping system, in accordance with embodiments of the present disclosure.
FIG. 1B is a schematic view of a mapping probe for use in association with embodiments of the system of FIG. 1A, in accordance with embodiments of the present disclosure.
FIG. 2 is a block diagram depicting an illustrative processing unit for use with a mapping system, in accordance with embodiments of the present disclosure.
FIG. 3 is a flow diagram depicting an illustrative process for generating a cardiac map, in accordance with embodiments of the present disclosure.
FIG. 4 is a flow diagram depicting an illustrative method for facilitating display of cardiac mapping information, in accordance with embodiments of the present disclosure.
FIG. 5 is a flow diagram depicting another illustrative method for facilitating display of cardiac mapping information, in accordance with embodiments of the present disclosure.
FIG. 6 is a flow diagram depicting another illustrative method for facilitating display of cardiac mapping information, in accordance with embodiments of the present disclosure.

While the disclosed subject matter is amenable to various modifications and alternative forms, specific embodiments have been shown by way of example in the drawings and are described in detail below. The intention, however, is not to limit the disclosure to the particular embodiments described. On the contrary, the disclosure is intended to cover all modifications, equivalents, and alternatives falling within the scope of the invention as defined by the appended claims.

As the terms are used herein with respect to ranges of measurements (such as those disclosed immediately above), "about" and "approximately" may be used, interchangeably, to refer to a measurement that includes the stated measurement and that also includes any measurements that are reasonably close to the stated measurement, but that may differ by a reasonably small amount such as will be understood, and readily ascertained, by individuals having ordinary skill in the relevant arts to be attributable to measurement error, differences in measurement and/or manufacturing equipment calibration, human error in reading and/or setting measurements, adjustments made to optimize performance and/or structural parameters in view of differences in measurements associated with other components, particular implementation scenarios, imprecise adjustment and/or manipulation of objects by a person or machine, and/or the like.

Although the term "block" may be used herein to connote different elements illustratively employed, the term should not be interpreted as implying any requirement of, or particular order among or between, various steps disclosed herein unless and except when explicitly referring to the order of individual steps.

### DETAILED DESCRIPTION

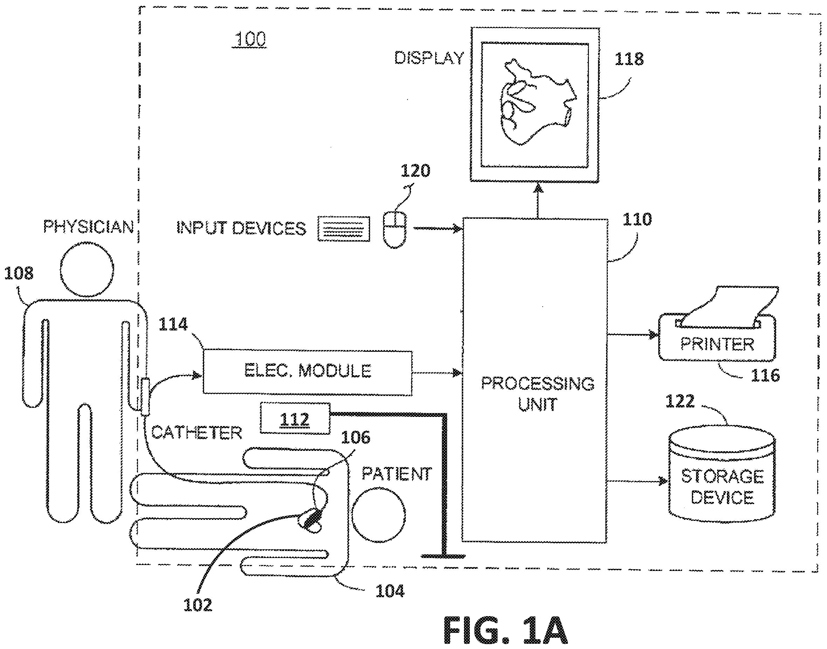

FIG. 1A shows a schematic diagram of an exemplary embodiment of an electro-anatomical mapping system 100, in accordance with embodiments of the disclosure. The mapping system 100 may be used for mapping cardiac structures 102 of a patient 104, and may be operated by one or more users 106, such as a physician and/or a technician. Throughout this disclosure, the term "cardiac structure" can mean any portion of a subject's heart and/or adjacent features such as, for example, an endocardium, an epicardium, an entire heart, a heart chamber, a portion of a heart chamber, a valve, a coronary sinus and/or its tributaries, a portion of a coronary sinus and/or a portion of its tributaries, a pulmonary artery, other surrounding vasculature and/or the like. As such, while the embodiments discussed herein are primarily discussed in relation to endocardium mapping, the embodiments may also be used in epicardium mapping, as well. While this disclosure discusses using the mapping system to map cardiac structures 102, embodiments of the mapping system 100 can also, or alternatively, be used to map other organs and biological tissue including, but not limited to, kidneys, lungs, brains, gall bladders, livers, spleens and intestines.

As shown in FIG. 1A, the mapping system 100 includes one or more catheters 106, each having one or more electrodes situated at or toward the distal end of the catheter 106 and/or along a body of the catheter 106. The one or more catheters 106 may be situated in or near a cardiac structure 102 of the patient 104. The system 100 may be configured to obtain intracardiac electrogram (EGM) signals from the one or more electrodes on one or more of the catheters 106. In embodiments, the one or more catheters 106 include one or more reference catheters, where each of the reference catheters includes one or more electrodes and may be secured in place in a stable position in or near the cardiac structure 102 or other structure being mapped. In embodiments, the one or more catheters 106 may include up to five reference catheters, each having one or more electrodes and being secured in place in a stable position in or near the structure being mapped. In some embodiments, the one or more reference catheters include at least one coronary sinus catheter.

In embodiments, the catheters 106 may include one or more mapping probes having one or more electrodes. For example, a mapping probe may include multiple spatially distributed electrodes disposed at a distal end of a flexible catheter body. During a cardiac mapping procedure, a mapping probe is displaced to multiple locations within the heart chamber into which the mapping probe is inserted. In embodiments, the distal end of the mapping probe is fitted with multiple electrodes spread somewhat uniformly over a distal end of the catheter. For example, the electrodes may be mounted on the mapping probe following a 3D olive shape, a basket shape, and/or the like. The electrodes are mounted on a device capable of deploying the electrodes into the desired shape while inside the heart, and retracting the electrodes when the catheter is removed from the heart. To allow deployment into a 3D shape in the heart, electrodes may be mounted on a balloon, shape memory material such as Nitinol, actuable hinged structure, and/or the like.

At each of the locations to which the mapping probe is moved, the multiple electrodes acquire cardiac electrical signals. The cardiac electrical signals may be sensed, for example, on the endocardial surface of the heart and/or in a heart chamber at a point away from the surface. As used herein, a mapped representation of a cardiac electrical signal feature that is sensed at a point inside a heart chamber is referred to as being below the endocardial surface. That is, for example, because the cardiac map includes an anatomical shell that represents an endocardial surface and, from the perspective of the viewer, a point that is located inside the chamber, and not on the endocardial surface, would appear to be "below" the mapped surface.

As the term is used herein, a sensed cardiac electrical signal may refer to one or more sensed signals. Each cardiac electrical signal may include a number of intracardiac electrograms (EGMs) sensed within a patient's heart, and may include any number of features that may be ascertained by aspects of the system 100. Examples of cardiac electrical signal features include, but are not limited to, activation times, minimum voltage values, maximum voltage values, maximum negative time-derivatives of voltages, instantaneous potentials, voltage amplitudes, dominant frequencies, peak-to-peak voltages, and/or the like. A cardiac electrical signal feature may refer to one or more features extracted from one or more cardiac electrical signals, derived from one or more features that are extracted from one or more cardiac electrical signals, and/or the like. Additionally, a representation, on a cardiac and/or a surface map, of a cardiac electrical signal feature may represent one or more cardiac electrical signal features, an interpolation of a number of cardiac electrical signal features, and/or the like.

Each cardiac signal also may be associated with a set of respective position coordinates that corresponds to the location at which the cardiac electrical signal was sensed. Each of the respective position coordinates for the sensed cardiac signals may include three-dimensional Cartesian coordinates, polar coordinates, and/or the like. In embodiments, other coordinate systems can be used. In embodiments, an arbitrary origin is used and the respective position coordinates refer to positions in space relative to the arbitrary origin. Since, in embodiments, the cardiac signals may be sensed on the endocardial surface, in the chamber enclosed by the endocardial surface and outside the endocardial surface, the respective position coordinates may be on the endocardial surface of the patient's heart, below the endocardial surface and/or on the epicardial surface.

Consequently, reconstructing and presenting to a user 108 (such as a physician and/or technician) physiological data pertaining to cardiac electrical activity may be based on information acquired at multiple locations, thereby providing a more accurate and faithful reconstruction of physiological behavior of the endocardium surface. In embodiments that include one or more mapping probes, at least one of the mapping probes may be displaced to multiple locations within the heart, where the acquisition of cardiac electrical signals at multiple probe locations in the heart chamber enables the one or more mapping probes to effectively act as a "mega-catheter" whose effective number of electrodes and electrode span is proportional to the product of the number of locations in which signal acquisition is performed and the number of electrodes on the one or more mapping probes. In embodiments, the one or more mapping probes may be configured for contact mapping, near-contact mapping, and/or non-contact mapping.

To enhance the quality of the reconstructed physiological information at the endocardium surface, in some embodiments a mapping probe may be moved to more than three locations (for example, more than 5, 10, or even 50 locations) within the heart chamber. Further, the spatial range over which the mapping probe is moved may be larger than one third (1/3) of the diameter of the heart cavity (for example, larger than 35%, 40%, 50% or even 60% of the diameter of the heart cavity). Additionally, in some embodiments the reconstructed physiological information is computed based on signals measured over several heart beats, either at a single location within the heart chamber or over several locations. In circumstances where the reconstructed physiological information is based on multiple measurements over several heart beats, the measurements may be synchronized with one another so that the measurement are performed at approximately the same phase of the heart cycle. The signal measurements over multiple beats may be synchronized based on features detected from physiological data such as surface ECG and/or intracardiac EGMs.

The mapping system 100 further includes a processing unit 110 which performs several of the operations pertaining to the mapping procedure, including the reconstruction procedure to determine the physiological information at the endocardium surface (e.g., as described above) and/or within a heart chamber. The processing unit 110 also may perform a catheter registration procedure. The processing unit 110 may be, include, or be included in, an electrical processor, a software processor, a general purpose microprocessor and/or a special purpose microprocessor, and may include a sole processor or multiple processors or cores.

The location of a catheter 106 inserted into the heart chamber can be determined using a sensing and tracking system 112 that provides the 3D spatial coordinates of the catheter 106 and/or its multiple electrodes with respect to the catheter's 106 coordinate system as established by the sensing and tracking system 112. Embodiments of the system 100 use a hybrid location technology that combines impedance location with magnetic location technology. This combination may enable the system 100 to accurately track mapping probes and/or other catheters 106 that are connected to the system 100. Magnetic location technology uses magnetic fields generated by a localization generator positioned under the patient table to track catheters with magnetic sensors. Impedance location technology may be used to track catheters that may not be equipped with a magnetic location sensor, and may utilize surface ECG patches.

In embodiments, to perform a mapping procedure and reconstruct physiological information on the endocardium surface, the processing unit 110 may align the coordinate system of a mapping probe (e.g., catheter 106) with the endocardium surface's coordinate system. The processing unit 110 (or some other processing component of the system 100) may determine a coordinate system transformation function that transforms the 3D spatial coordinates of the catheter's 106 locations into coordinates expressed in terms of the endocardium surface's coordinate system, and/or vice-versa. In embodiments, such a transformation may not be necessary. The processing unit 110 also may perform post-processing operations on the physiological information to extract and display useful features of the information to the user 108 of the system 100 and/or other persons (e.g., a physician).

According to embodiments, the signals acquired by the multiple electrodes of a catheter 106 (e.g., a mapping probe) are passed to the processing unit 110 via an electrical module 114, which may include, for example, a signal conditioning component. The electrical module 114 may be configured to receive the signals communicated from the catheter 106 and perform signal enhancement operations on the signals before they are forwarded to the processing unit 110. The electrical module 114 may include signal conditioning hardware, software, and/or firmware that may be used to amplify, filter and/or sample intracardiac potential measured by one or more electrodes. In embodiments, the electrical module 114 may also be configured to receive signals from any number of other catheters, provide signals to catheters, and/or the like.

The cardiac electrical signals typically have a maximum amplitude of 60mV, with a mean of a few millivolts. In embodiments, the signals are bandpass filtered in a frequency range (e.g., 0.5-500Hz) and sampled with analog to digital converters (e.g., with 15-bit resolution at 1 kHz). To avoid interference with electrical equipment in the room, the signals may be filtered to remove the frequency corresponding to the power supply (e.g., 60 Hz). Other types of signal processing operations such as spectral equalization, automatic gain control, etc. may also take place. For example, in embodiments, the cardiac electrical signals may be unipolar signals, measured relative to a reference (which may be a virtual reference) such as, for example, a coronary sinus catheter or Wilson's Central Terminal (WCT), from which the signal processing operations may compute differences to generate bipolar signals. The signals may be otherwise processed (e.g., filtered, sampled, etc.) before and/or after generating the bipolar signals. The resultant processed signals are forwarded by the electrical module 114 to the processing unit 110 for further processing.

As further shown in FIG. 1A, the cardiac mapping system 100 also may include peripheral devices such as a printer 116, a display device 118 and/or input devices 120, all of which may be interconnected to the processing unit 110. Additionally, the mapping system 100 includes storage device 122 that may be used to store data acquired by the various interconnected modules, including the volumetric images, raw data measured by electrodes and/or the resultant endocardium representation computed therefrom, the partially computed transformations used to expedite the mapping procedures, the reconstructed physiological information corresponding to the endocardium surface, and/or the like. According to embodiments, the storage device may also, or alternatively, be used to store computer-executable instructions for facilitating aspects of embodiments of procedures described herein.

The illustrative mapping system 100 shown in FIG. 1A is not intended to suggest any limitation as to the scope of use or functionality of embodiments of the present disclosure. Neither should the illustrative mapping system 100 be interpreted as having any dependency or requirement related to any single component or combination of components illustrated therein. Additionally, various components depicted in FIG. 1A may be, in embodiments, integrated with various ones of the other components depicted therein (and/or components not illustrated), all of which are considered to be within the ambit of the present disclosure. For example, the electrical module 114 may be integrated with the processing unit 110 and/or the storage device 120 may be integrated with the processing unit 110.

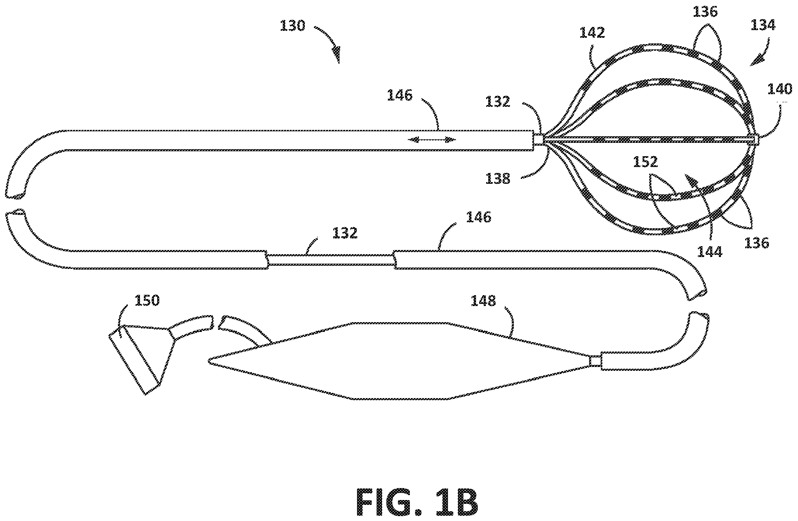

FIG. 1B is a schematic view of a mapping probe 130, which may, for example, be used in association with embodiments of the system 100 of FIG. 1A, in accordance with embodiments of the disclosure. The mapping probe 130 has a flexible catheter body 132, the distal end of which carries the three-dimensional basket structure 134 that includes the mapping electrodes 136. As stated above, the mapping electrodes 136 sense cardiac electrical signals; and the sensed signals are sent to a processing unit 110, via a wired and/or wireless connection. The processing unit 110 processes the sensed signals and a cardiac map is created. The types of cardiac maps created can include, but are not limited to, the following: a voltage map, an activation map, a fractionation map, a velocity map, and/or the like.

The basket structure 134 comprises a base member 138 and an end cap 140 between which flexible splines 142 generally extend in a circumferentially spaced relationship. In embodiments, the basket structure 134 takes the form of a basket defining an open interior space 144. In embodiments, the splines 142 are made of a resilient inert material, such as Nitinol metal or silicone rubber, and are connected between the base member 138 and the end cap 140 in a resilient, pre-tensed condition, to bend and conform to the tissue surface they contact. In other embodiments, such as the catheters described in U.S. Pat. Nos. 8,103,327, 8,447,377, and 8,755,861, which are entitled "CARDIAC MAPPING CATHETER" the splines of the catheter may not bend and conform to the tissue surface they contact. In the illustrated embodiments, eight splines 142 form the three-dimensional structure 134. Additional or fewer splines 142 could be used in other embodiments, and the three-dimensional structure 134 may be configured according to any number of different shapes such as, for example, generally spherical shapes, generally elliptical shapes, generally tear-drop shapes, and/or the like. As illustrated, each spline 142 carries eight mapping electrodes 136. Additional or fewer mapping electrodes 136 could be disposed on each spline 142 in other embodiments of the three-dimensional structure 134. In the illustrated embodiments, the three-dimensional structure 134 is relatively small (e.g., 40 mm or less in diameter). In embodiments, the three-dimensional structure 134 is larger (e.g., 40 mm in diameter or greater).

In embodiments, a slidable sheath 146 is movable along the major axis of the catheter body 132. Moving the sheath 146 forward (i.e., toward the distal end) causes the sheath 146 to move over the three-dimensional structure 134, thereby collapsing the structure 134 into a compact, low profile condition suitable for introduction into an interior space, such as, for example, into the heart. In contrast, moving the sheath 146 rearward (i.e., toward the proximal end) exposes the three-dimensional structure 134, allowing the structure 134 to elastically expand and assume the pre-tensed position illustrated in FIG. 1B. Further details of embodiments of the three-dimensional structure 134 are disclosed, for example, in U.S. Pat. No. 5,647,870, entitled "MULTIPLE ELECTRODE SUPPORT STRUCTURES."

Additionally, further details of mapping catheters that may be used in association with embodiments of the system 100 of FIG. 1A, in accordance with embodiments of the disclosure, are disclosed, for example, in U.S. Pat. Nos. 8,103,327, 8,447,377 and 8,755,861.

In embodiments where the mapping probe 130 uses a wired connection, a signal wire (not shown) may be electrically coupled to each mapping electrode 136. The wires may extend through the body 132 of the mapping catheter 130 into a handle 148, in which they may be coupled to an external connector 150, which may be, for example, a multiple pin connector. The connector 150 electrically couples the mapping electrodes 136 to the processing system 110. Further details on mapping systems and methods for processing signals generated by mapping catheters are discussed, for example, in U.S. Pat. No. 6,070,094, entitled "SYSTEMS AND METHODS FOR GUIDING MOVABLE ELECTRODE ELEMENTS WITHIN MULTIPLE-ELECTRODE STRUCTURE;" U.S. Pat. No. 6,233,491, entitled "CARDIAC MAPPING AND ABLATION SYSTEMS;" and U.S. Pat. No. 6,735,465, entitled "SYSTEMS AND PROCESSES FOR REFINING A REGISTERED MAP OF A BODY CAVITY."

Additionally, further details of mapping systems and methods for processing signals generated by mapping catheters are discussed, for example, in U.S. Pat. No. 8,103,338, entitled "Impedance Based Anatomy Generation;" U.S. Pat. No. 8,615,287, entitled "Catheter Tracking and Endocardium Representation Generation;" and U.S. Pat. No. 9,107,599, entitled "Electroanatomical Mapping."

It is noted that other electrode structures could be deployed on the distal end of a mapping probe 130. It is further noted that the multiple mapping electrodes 136 may be disposed on more than one structure, rather than, for example, the single mapping probe 130 illustrated in FIG. 1B. For example, if mapping within the left atrium with multiple mapping structures, an arrangement comprising a coronary sinus catheter carrying multiple mapping electrodes and a basket catheter carrying multiple mapping electrodes positioned in the left atrium may be used. As another example, if mapping within the right atrium with multiple mapping structures, an arrangement comprising a decapolar catheter carrying multiple mapping electrodes for positioning in the coronary sinus, and a loop catheter carrying multiple mapping electrodes for positioning around the tricuspid annulus may be used. Additionally, in embodiments, the mapping electrodes 136 may be separated from one another by insulated gaps 152.

Although the mapping electrodes 136 have been described as being carried by dedicated mapping probes, such as the mapping probe 130, the mapping electrodes 136 may be carried on non-mapping dedicated probes or multifunction probes. For example, an ablation catheter can be configured to include one or more mapping electrodes.

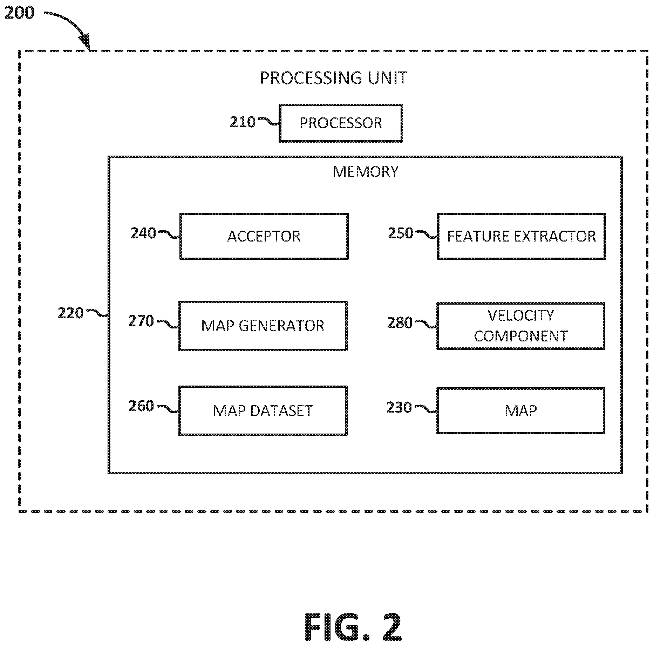

FIG. 2 is a block diagram of an illustrative processing unit 200, in accordance with embodiments of the disclosure. The processing unit 200 may be, be similar to, include, or be included in the processing unit 110 depicted in FIG. 1A. As shown in FIG. 2, the processing unit 200 may be implemented on a computing device that includes a processor 210 and a memory 220. Although the processing unit 200 is referred to herein in the singular, the processing unit 200 may be implemented in multiple instances (e.g., as a server cluster), distributed across multiple computing devices, instantiated within multiple virtual machines, and/or the like. One or more components for facilitating cardiac mapping may be stored in the memory 220. In embodiments, the processor 210 may be configured to instantiate the one or more components to generate a cardiac map 230, which may be stored in the memory 220.

As is further depicted in FIG. 2, the processing unit 200 may include an acceptor 240 configured to receive cardiac electrical signals from a mapping probe (e.g., the catheter 106 depicted in FIG. 1A, and/or the mapping probe 130 depicted in FIG. 1B). The sensed cardiac electrical signals may include a number of intracardiac electrograms (EGMs) sensed within a patient's heart. The acceptor 240 may also receive an indication of a measurement location corresponding to each of the electrical signals. In embodiments, the acceptor 240 may be configured to determine whether to accept the cardiac electrical signals that have been received. The acceptor 240 may utilize any number of different components and/or techniques to determine which electrical signals or beats to accept, such as filtering, beat matching, morphology analysis, positional information (e.g., catheter motion), respiration gating, and/or the like. In embodiments, for example, the acceptor 240 may accept and reject cardiac electrical signals (e.g., EGMS), signal features, beats, and/or the like, based on velocity information, as described in further detail below.

The accepted cardiac electrical signals are received by a feature extractor 250 that is configured to extract one or more features from each of the cardiac electrical signals. According to embodiments, the features may include, for example, features, extracted from the cardiac electrical signals and/or derived from other features, such as, for example, activation times, minimum voltage values, maximum voltage values, maximum negative time-derivatives of voltages, instantaneous potentials, voltage amplitudes, dominant frequencies, peak-to-peak voltages, and/or the like. The acceptor 240 and/or feature extractor 250 may generate, from the acquired cardiac electrical signals, a map dataset 260.

As shown in FIG. 2, the processing unit 200 includes a map generator 270 that is configured to generate, and facilitate presentation of, a map 230 corresponding to a cardiac structure based on the map dataset 260. For example, the map generator 270 may be configured to facilitate presentation of the map 230 corresponding to the cardiac structure by performing interpolation, extrapolation and/or fitting based on cardiac signal features, velocity information, and/or the like. In embodiments, the map 230 may include a voltage map, an activation map, a fractionation map, a velocity map, a confidence map, and/or the like.

As is further shown in FIG. 2, the processing unit 200 includes a velocity component 280 that may be configured to determine one or more conduction velocities associated with an activation propagation. For example, in embodiments, the velocity component 280 may be configured to determine a number of conduction velocities corresponding to signal features extracted by the feature extractor 250. In this manner, the map generator 270 may be configured to generate the cardiac map 230 based on extracted signal features and conduction velocities determined by the velocity component 280. In other embodiments, the determined conduction velocities may correspond to map features generated by the map generator 270 rather than individual signal features extracted by the feature extractor 250. In embodiments, the map 230 may include an anatomical shell and a set of annotations representing at least a portion of the extracted signal features.

In embodiments, for example, the processing unit 100 (e.g., using the velocity component 280 and/or the map generator 270) may be configured to determine a set of conduction velocities, of a plurality of conduction velocities, that satisfy a condition, where the set of annotations in the map 230 includes a representation of the signal features that correspond to one of the set of conduction velocities that satisfy the condition.

In embodiments, the velocity component 280 may be configured to determine a conduction velocity based on a "pre-filter" method or a "post-filter" method.

In embodiments, pre-filtering conduction velocities may include determining a propagation velocity using electrograms from a single cycle (or "beat"). Specifically, the relative activation times of nearby electrodes of a mapping catheter, in combination with their relative locations of the electrodes, can be used to estimate a local conduction velocity. In embodiments, the set of annotations in the map 230 can include a representation of the signal features that corresponds to the pre-filtered conduction velocities that satisfy a condition. Pre-filtering may be useful for rejecting electrograms (or entire beats) from the map based on the estimated local conduction velocities.

In embodiments, post-filtering conduction velocities may include building an activation map and estimating the velocity at each map vertex by determining a gradient at a map vertex using the activation values of nearby vertices. In embodiments, the set of annotations in the map 230 can include a representation of the signal features at map locations for which the post-filtered conduction velocities satisfy a condition.

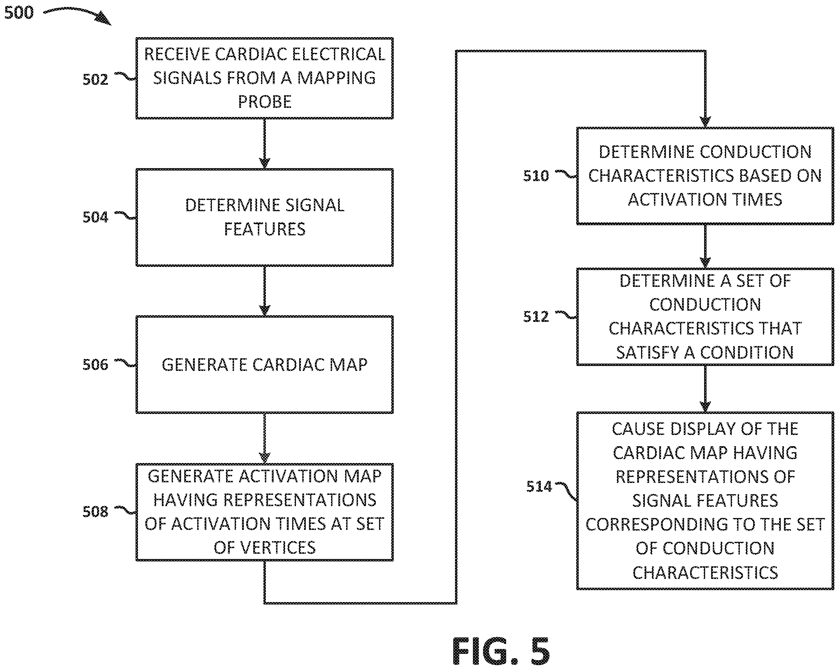

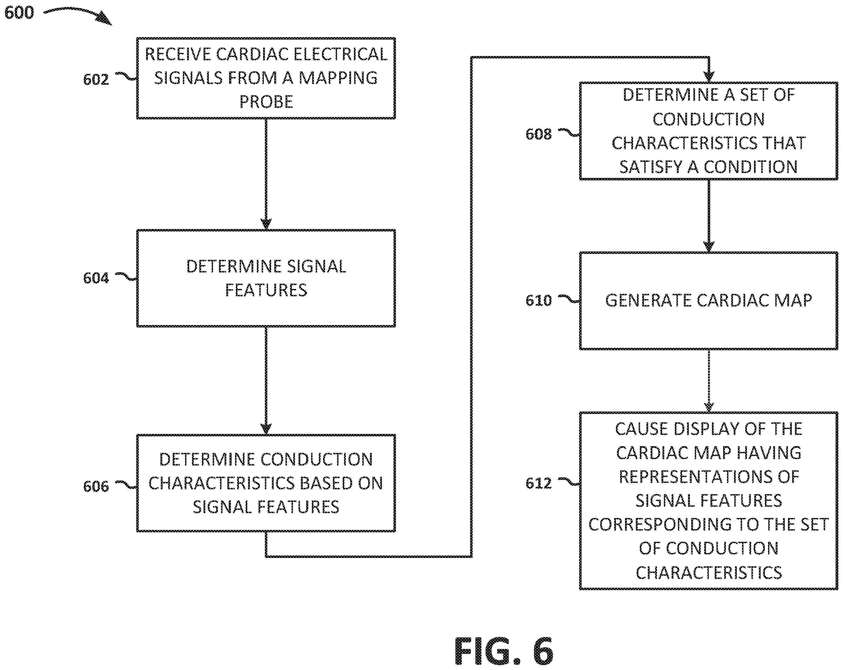

Post-filtering and pre-filtering are explained in more detail below in FIGS. 5 and 6, respectively.

The illustrative processing unit 200 shown in FIG. 2 is not intended to suggest any limitation as to the scope of use or functionality of embodiments of the present disclosure. Neither should the illustrative processing unit 200 be interpreted as having any dependency or requirement related to any single component or combination of components illustrated therein. Additionally, any one or more of the components depicted in FIG. 2 may be, in embodiments, integrated with various ones of the other components depicted therein (and/or components not illustrated), all of which are considered to be within the ambit of the present disclosure. For example, the acceptor 240 may be integrated with the feature extractor 250. Additionally, the processing unit 200 may (alone and/or in combination with other components of the system 100 depicted in FIG. 1, and/or other components not illustrated) perform any number of different functions and/or processes associated with cardiac mapping (e.g., triggering, blanking, field mapping, etc.) such as, for example, those described in U.S. Patent 8,428,700, entitled "ELECTROANATOMICAL MAPPING;" U.S. Patent 8,948,837, entitled "ELECTROANATOMICAL MAPPING;" U.S. Patent 8,615,287, entitled "CATHETER TRACKING AND ENDOCARDIUM REPRESENTATION GENERATION;" U.S. Patent Publication 2015/0065836, entitled "ESTIMATING THE PREVALENCE OF ACTIVATION PATTERNS IN DATA SEGMENTS DURING ELECTROPHYSIOLOGY MAPPING;" U.S. Patent 6,070,094, entitled "SYSTEMS AND METHODS FOR GUIDING MOVABLE ELECTRODE ELEMENTS WITHIN MULTIPLE-ELECTRODE STRUCTURE;" U.S. Patent 6,233,491, entitled "CARDIAC MAPPING AND ABLATION SYSTEMS;" and U.S. Patent 6,735,465, entitled "SYSTEMS AND PROCESSES FOR REFINING A REGISTERED MAP OF A BODY CAVITY."

According to embodiments, various components of the mapping system 100, illustrated in FIGS. 1A and 1B, and/or the processing unit 200, illustrated in FIG. 2, may be implemented on one or more computing devices. A computing device may include any type of computing device suitable for implementing embodiments of the disclosure. Examples of computing devices include specialized computing devices or general-purpose computing devices such "workstations," "servers," "laptops," "desktops," "tablet computers," "hand-held devices," "general-purpose graphics processing units (GPGPUs)," and the like, all of which are contemplated within the scope of FIGS. 1A and 2 with reference to various components of the system 100 and/or processing unit 200.

In embodiments, a computing device includes a bus that, directly and/or indirectly, couples the following devices: a processor, a memory, an input/output (I/O) port, an I/O component, and a power supply. Any number of additional components, different components, and/or combinations of components may also be included in the computing device. The bus represents what may be one or more busses (such as, for example, an address bus, data bus, or combination thereof). Similarly, in embodiments, the computing device may include a number of processors, a number of memory components, a number of I/O ports, a number of I/O components, and/or a number of power supplies. Additionally any number of these components, or combinations thereof, may be distributed and/or duplicated across a number of computing devices.

In embodiments, memory (e.g., the storage device 120 depicted in FIG. 1A, and/or the memory 220 depicted in FIG. 2) includes computer-readable media in the form of volatile and/or nonvolatile memory and may be removable, nonremovable, or a combination thereof. Media examples include Random Access Memory (RAM); Read Only Memory (ROM); Electronically Erasable Programmable Read Only Memory (EEPROM); flash memory; optical or holographic media; magnetic cassettes, magnetic tape, magnetic disk storage or other magnetic storage devices; data transmissions; and/or any other medium that can be used to store information and can be accessed by a computing device such as, for example, quantum state memory, and/or the like. In embodiments, the memory 120 and/or 220 stores computer-executable instructions for causing a processor (e.g., the processing unit 110 depicted in FIG. 1 and/or the processor 210 depicted in FIG. 2) to implement aspects of embodiments of system components discussed herein and/or to perform aspects of embodiments of methods and procedures discussed herein.

Computer-executable instructions may include, for example, computer code, machine-useable instructions, and the like such as, for example, program components capable of being executed by one or more processors associated with a computing device. Examples of such program components include the map 230, the acceptor 240, the feature extractor 250, the map dataset 260, the map generator 270, and the velocity component 280. Program components may be programmed using any number of different programming environments, including various languages, development kits, frameworks, and/or the like. Some or all of the functionality contemplated herein may also, or alternatively, be implemented in hardware and/or firmware.

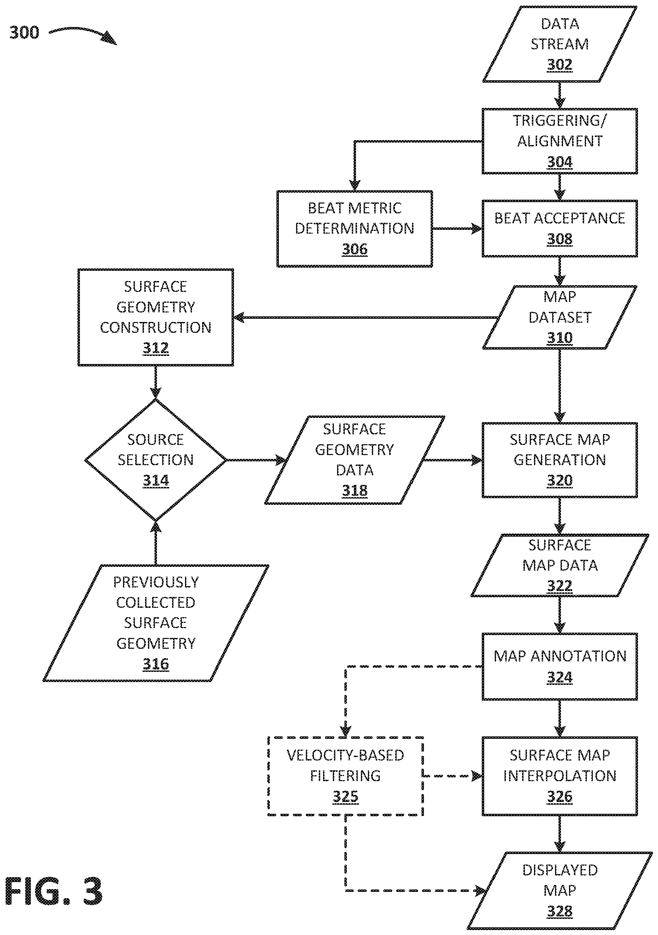

FIG. 3 is a flow diagram of an illustrative process 300 for automated electro-anatomical mapping, in accordance with embodiments of the disclosure. Aspects of embodiments of the method 300 may be performed, for example, by a processing unit (e.g., the processing unit 110 depicted in FIG. 1A, and/or the processing unit 200 depicted in FIG. 2). A data stream 302 containing multiple signals is first input into the system (e.g., the mapping system 100 depicted in FIG. 1). During the automated electro-anatomical mapping process, a data stream 302 provides a collection of physiological and non-physiological signals that serve as inputs to the mapping process. The signals may be collected directly by the mapping system, and/or obtained from another system using an analog or digital interface. The data stream 302 may include cardiac electrical signals such as unipolar and/or bipolar intracardiac electrograms (EGMs), surface electrocardiograms (ECGs), electrode location information originating from one or more of a variety of methodologies (magnetic, impedance, ultrasound, real time MRI, etc.), tissue proximity information, catheter force and/or contact information obtained from one or more of a variety of methodologies (force spring sensing, piezo-electric sensing, optical sensing etc.), catheter tip and/or tissue temperature, acoustic information, catheter electrical coupling information, catheter deployment shape information, electrode properties, respiration phase, blood pressure, other physiological information, and/or the like.

For the generation of specific types of maps, one or more signals may be used as one or more references, during a triggering/alignment process 304, to trigger and align the data stream 302 relative to the cardiac, other biological cycle and/or an asynchronous system clock resulting in beat datasets. Additionally, for each incoming beat dataset, a number of beat metrics are computed during a beat metric determination process 306. Beat metrics may be computed using information from a single signal, spanning multiple signals within the same beat and/or from signals spanning multiple beats. The beat metrics provide multiple types of information on the quality of the specific beat dataset and/or likelihood that the beat data is good for inclusion in the map dataset (e.g., the map dataset 260 depicted in FIG. 2).

A beat acceptance process 308 aggregates the criteria and determines which beat datasets will make up the map dataset 310. As explained herein, in embodiments, beat datasets, which may include whole beats, whole cardiac electrical signals (e.g., EGMs), and/or extracted features, may be accepted and/or rejected during the beat acceptance process 308 based on determined conduction velocities (e.g., in a pre-filtering process).

Surface geometry data may be generated concurrently during the same data acquisition process using identical and/or different triggering and/or beat acceptance metrics employing a surface geometry construction process 312. This process constructs surface geometry using data such as electrode locations and catheter shape contained in the data stream. Additionally, or alternatively, previously collected surface geometry 316 may be used as an input to surface geometry data 318. Such geometry may have been collected previously in the same procedure using a different map dataset, and/or using a different modality such as CT, MRI, ultrasound, rotational angiography, and/or the like, and registered to the catheter locating system. The system performs a source selection process 314, in which it selects the source of the surface geometry data and provides surface geometry data 318 to a surface map generation process 320. The surface map generation process 320 is employed to generate surface map data 322 from the map dataset 320 and surface geometry data 318.

The surface geometry construction algorithm generates the anatomical surface on which the electroanatomical map is displayed. Surface geometry can be constructed, for example, using aspects of a system as described U.S. Patent Application Serial No. 12/437,794, entitled "Impedance Based Anatomy Generation" and filed on May 8, 2008; and/or U.S. Patent 8,948,837, entitled "Electroanatomical Mapping" and issued on February 3, 2015. Additionally, or alternatively, an anatomical shell can be constructed by the processing unit by fitting a surface on electrode locations that are determined either by the user or automatically to be on the surface of the chamber. In addition, a surface can be fit on the outermost electrode and/or catheter locations within the chamber.

As described, the map dataset 310 from which the surface is constructed can employ identical or different beat acceptance criteria from those used for electrical and other types of maps. The map dataset 310 for surface geometry construction can be collected concurrently with electrical data or separately. Surface geometry can be represented as a mesh containing a collection of vertices (points) and the connectivity between them (e.g. triangles). Alternatively, surface geometry can be represented by different functions such as higher order meshes, non-uniform rational basis splines (NURBS), and/or curvilinear shapes.

The generation process 320 generates surface map data 322. The surface map data 322 may provide information on cardiac electrical excitation, cardiac motion, tissue proximity information, tissue impedance information, force information, and/or any other collected information desirable to the clinician. The combination of map dataset 320 and surface geometry data 318 allows for surface map generation. The surface map is a collection of values or waveforms (e.g., EGMs) on the surface of the chamber of interest, whereas the map dataset can contain data that is not on the cardiac surface. One approach for processing the map dataset 320 and surface geometry data 318 to obtain a surface map dataset 322 is described in US 7,515,954, entitled "NON-CONTACT CARDIAC MAPPING, INCLUDING MOVING CATHETER AND MULTI-BEAT INTEGRATION" and filed June 13, 2006.

Alternatively, or in combination with the method above, an algorithm that applies acceptance criteria to individual electrodes can be employed. For example, electrode locations exceeding a set distance (e.g., 3mm) from surface geometry can be rejected. Another algorithm can incorporate tissue proximity information using impedance for inclusion in the surface map data. In this case only electrode location whose proximity value is less than 3mm might be included. Additional metrics of the underlying data can also be used for this purpose. For example, EGM properties similar to beat metrics such as, for example, conduction velocities, can be assessed on a per electrode basis. In this case metrics such as far field overlap and/or EGM consistency can be used. It should be understood that variations on the method to project points from the map dataset 320 to the surface and/or to select appropriate points can exist.

Once obtained, the surface map data 322 may be further processed to annotate desired features from the underlying data, a process defined as surface map annotation 324. Once data is collected into surface map data 322, attributes relating to the collected data may be automatically presented to the user. These attributes can be automatically determined and applied to the data by the computer system and are referred to herein as annotations. Exemplary annotations include activation time, the presence of double activation or fractionation, voltage amplitude, spectral content, and/or the like. Due to the abundance of data available in automated mapping (e.g., mapping completed by the computer system with minimal human input related to the incoming data), it is not practical for the operator to review and annotate data manually. However, human input can be a valuable addition to the data, and so when user input is provided it is necessary for the computer system to automatically propagate and apply it to more than one data point at a time. Further details of annotating desired features are disclosed, for example, in U.S. Pat. No. 8,948,837, entitled "ELECTROANATOMICAL MAPPING."

It may be possible to use the computer system to automatically annotate activation time, voltage, and other characteristics of individual EGMs. Activation time detection may use methods similar to those previously described to detect a trigger and can similarly benefit from the use of blanking and powered triggering operator. Desired annotations may include instantaneous potential, activation time, voltage amplitude, dominant frequency, a mean cycle length and/or other properties of the signal. Once computed, the annotations may be displayed superimposed on chamber geometry. In embodiments, the map may be post-filtered in a velocity-based filtering process 325. That is, for example, the system may generate an activation map and estimate the velocity at each vertex of the activation map by finding the gradient of nearby map values. Then, the cardiac map may be annotated only at locations where the velocity is within a certain range.

In embodiments, a gap-filling surface map interpolation may be employed 326. For example, in embodiments, a gap-filling interpolation may be employed where a distance between a point on the surface to a measured EGM exceeds a threshold. Displayed maps 328 can be computed and displayed separately, and/or overlaid on top of each other. As shown, the velocity-based filtering described above may alternatively be applied just before (or, e.g., as part of the process of) displaying the map 328. In embodiments, for example, they system may generate a velocity mask by generating a separate velocity map (e.g., based on a generated activation map) having conduction velocity values associated with vertices that correspond to the vertices of the generated and annotated cardiac map. When the map is displayed 328, only those portions of the map geometry and/or annotations are displayed that correspond to velocities that satisfy a particular condition.

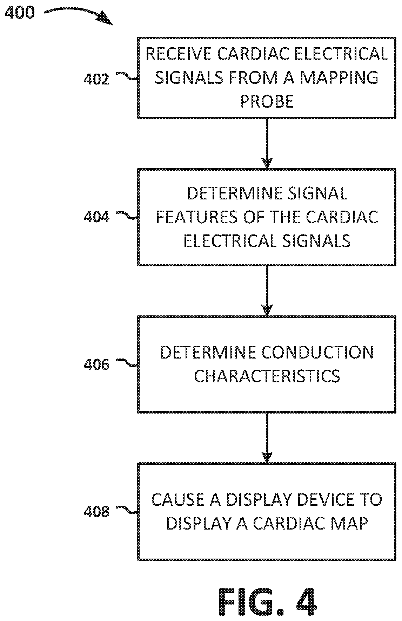

FIG. 4 is a flow diagram depicting an illustrative method 400 for facilitating display of cardiac mapping information, in accordance with embodiments of the disclosure. Embodiments of method 400 may be performed, in whole or in part, by a mapping system (e.g., the mapping system 100 depicted in FIG. 1A). Embodiments of the method 400 include receiving, from a mapping probe, a number of cardiac electrical signals (block 402) and determining a number of signal features, each signal feature including a feature of one of the sensed cardiac signals (block 404). Each of the sensed cardiac electrical signals (and, accordingly, features of each signal) corresponds to a respective point (e.g., location in space) where the cardiac electrical signal was sensed.

The sensed cardiac electrical signals can include a number of intracardiac EGMs. Examples of features of the cardiac electrical signals include, but are not limited to, activation times, minimum voltage values, maximum voltage values, maximum negative time-derivatives of voltages, instantaneous potentials, voltage amplitudes, dominant frequencies, peak-to-peak voltages, and/or the like. Each of the respective points at which a cardiac electrical signal is sensed has a corresponding set of three-dimensional position coordinates. For example, the position coordinates of the points may be represented in Cartesian coordinates. Other coordinate systems can be used, as well. In embodiments, an arbitrary origin is used and the respective position coordinates are defined with respect to the arbitrary origin. In embodiments, the point corresponding to each sensed cardiac electrical signal may be located on the endocardial surface of the heart and/or below the endocardial surface of the heart.

Embodiments of the method 400 also include determining a number of conduction characteristics, each of the conduction characteristics based on one or more of the plurality of signal features (block 406). In embodiments, the conduction characteristics may be activation gradients or conduction velocities. A conduction characteristic may be "based on" one or more of the plurality of signal features according to the post-filter method and/or the pre-filter method described above with regard to FIG. 2. That is, the term "based on" implies derivation at some level, but is not meant to imply a direct calculation or other determination. Any number of calculations and/or other types of determinations, transformations, aggregations, and/or the like, may be interposed between a characteristic (e.g., a value, classification, etc.) and a fact upon which it is based. For example, a conduction characteristic may be determined by performing a calculation using one or more values (e.g., activation times, differences between activation times, etc.) associated with a map vertex, where those values may be derived from some other information (e.g., activation times may be features of a signal). In embodiments of the "post-filter" method, a conduction characteristic may be determined based on a difference between two activation times, each of which may actually be determined by aggregating information from multiple cardiac signals and/or signal features. As another example, conduction characteristics based on one or more signal features may include a conduction characteristic corresponding to a propagation vector for an activation signal that is determined using activation times corresponding to electrical signals sensed by three electrodes (i.e., a conduction characteristic determined using the pre-filter method). In addition to the description given above, each of these methods are described in more detail below in FIGS. 5 and 6, respectively.

Embodiments of the method 400 also include causing a display device to display, based on the conduction characteristics, a cardiac map of at least a portion of one or more cardiac structures (block 408). In embodiments, the cardiac map may be generated based, at least in part, on the cardiac electrical signal features and may include an anatomical shell and a representation of at least one of the signal features. In embodiments, the cardiac map may also be generated, at least in part, using any number of other signals, techniques, and/or the like. For example, embodiments may utilize impedance mapping techniques to generate one or more portions of the cardiac map such as, for example, an anatomical shell upon which electrical signal features are represented. In embodiments, a surface may be fitted on one or more of the points associated with the cardiac electrical signals to generate a shell representing the endocardial surface of the one or more cardiac structures. In embodiments, a surface may also be fitted on one or more of the points associated with the cardiac electrical signals to generate a shell representing an epicardium surface or other excitable cardiac tissue. In embodiments, one or more of the cardiac electrical signal features at the corresponding points can be included on the shell to generate a cardiac map of the one or more cardiac structures. For example, embodiments may include displaying annotations on the cardiac map that represent features, extracted from the cardiac electrical signals and/or derived from other features, such as, for example, activation times, minimum voltage values, maximum voltage values, maximum negative time-derivatives of voltages, instantaneous potentials, voltage amplitudes, dominant frequencies, peak-to-peak voltages, and/or the like.

FIG. 5 is a flow diagram depicting another illustrative method 500 for facilitating display of cardiac mapping information, in accordance with embodiments of the present disclosure. Embodiments of method 500 may be performed, in whole or in part, by a mapping system (e.g., the mapping system 100 depicted in FIG. 1A). Embodiments of the method 500 include receiving, from a mapping probe, a number of cardiac electrical signals (block 502) and determining a number of signal features, each signal feature including a feature of one of the sensed cardiac signals (block 504). Each of the sensed cardiac electrical signals (and, accordingly, features of each signal) corresponds to a respective point (e.g., location in space) where the cardiac electrical signal was sensed.

The sensed cardiac electrical signals can include a number of intracardiac EGMs. Examples of features of the cardiac electrical signals include, but are not limited to, activation times, minimum voltage values, maximum voltage values, maximum negative time-derivatives of voltages, instantaneous potentials, voltage amplitudes, dominant frequencies, peak-to-peak voltages, and/or the like. Each of the respective points at which a cardiac electrical signal is sensed has a corresponding set of three-dimensional position coordinates. For example, the position coordinates of the points may be represented in Cartesian coordinates. Other coordinate systems can be used, as well. In embodiments, an arbitrary origin is used and the respective position coordinates are defined with respect to the arbitrary origin. In embodiments, the point corresponding to each sensed cardiac electrical signal may be located on the endocardial surface of the heart and/or below the endocardial surface of the heart.

Embodiments of method 500 also include generating a cardiac map (block 506). In embodiments, the cardiac map may be generated based, at least in part, on the cardiac electrical signal features and may include an anatomical shell and a representation of at least one of the signal features. In embodiments, the cardiac map may also be generated, at least in part, using any number of other signals, techniques, and/or the like. For example, embodiments may utilize impedance mapping techniques to generate one or more portions of the cardiac map such as, for example, an anatomical shell upon which electrical signal features are represented. In embodiments, a surface may be fitted on one or more of the points associated with the cardiac electrical signals to generate a shell representing the endocardial surface of the one or more cardiac structures. In embodiments, a surface may also be fitted on one or more of the points associated with the cardiac electrical signals to generate a shell representing an epicardium surface or other excitable cardiac tissue. In embodiments, the surface geometry of the shell can be represented as a mesh containing a collection of vertices (points) and the connectivity between them (e.g. triangles). Alternatively, surface geometry can be represented by different functions such as higher order meshes, non-uniform rational basis splines (NURBS), and/or curvilinear shapes.

In embodiments, one or more of the cardiac electrical signal features at the corresponding points can be included on the shell to generate a cardiac map of the one or more cardiac structures. For example, embodiments may include displaying annotations on the cardiac map that represent features, extracted from the cardiac electrical signals and/or derived from other features, such as, for example, activation times, minimum voltage values, maximum voltage values, maximum negative time-derivatives of voltages, instantaneous potentials, voltage amplitudes, dominant frequencies, peak-to-peak voltages, and/or the like. If a cardiac map other than an activation map is produced as part of block 506, embodiments of method 500 also include generating an activation map having a set of vertices (block 508).

Embodiments of method 500 include determining conduction characteristics based on the activation times that are extracted from the cardiac electrical signals (block 510). As stated above, in embodiments, conduction characteristics may be activation gradients or conduction velocities. Determining conduction characteristics based on gradients of activation times may be referred to herein, interchangeably, as a "post-filter" method for determining conduction characteristics.

In embodiments, to determine conduction characteristics based on gradients of activation times, two or more activation time differences for each vertex of the activation map may be computed. The activation time differences of a vertex may be computed with respect to the activation times of surrounding vertices. Location differences of a vertex may also be computed with respect to the locations of surrounding vertices. Using the two or more activation time differences and the corresponding location differences, the activation time gradient for a vertex may be estimated using a least-squares fit. The term "least-squares gradient" may be used, interchangeably, to refer to calculating a single gradient from the two or more activation time differences and location differences using least-squares. The direction of the activation propagation will be the direction of the least-squares gradient. Furthermore, when comparing the least-squares gradient for two different vertices, the vertex with the least-squares gradient that has the larger magnitude will be the vertex that has slower conduction velocity. This is because the differences in activation times surrounding a first vertex that has slow propagation between the first vertex and neighboring vertices will be greater than the differences in activation times surrounding a second vertex that has fast propagation between the second vertex and neighboring vertices.

In embodiments, a conduction velocity for a vertex may be determined from the activation time gradient by calculating the reciprocal of the activation time gradient. In these embodiments, when comparing the least-squares gradient for two different vertices, the vertex with the least-squares gradient that has the smaller magnitude will be the vertex that has slower conduction velocity.

Embodiments of method 500 include determining a set of conduction characteristics that satisfy a condition (block 512). In the invention as claimed, the condition is selected based on a conduction velocity. For example, as described above, reentry of an activation signal, which may result in an arrhythmia, may be due to a slow pathway. As such, the condition for the conduction characteristics may be set to distinguish between a propagation speed that is typical of healthy cardiac tissue and a propagation speed that is typical of unhealthy cardiac tissue. To distinguish between propagation speeds, a range of conduction characteristic values that is typical of unhealthy cardiac tissue may satisfy the condition and/or conduction characteristic values that are above or below a threshold value may satisfy the condition.

For example, when the conduction characteristic are activation time gradients and when comparing the activation time gradient for two different vertices, the vertex with the activation time gradient that has the larger magnitude will be the vertex that has slower conduction velocity. Accordingly, in embodiments, the activation gradient may satisfy the condition when the activation gradient is no less than a threshold. And, conversely, when the conduction characteristics are conduction velocities and when comparing the conduction velocities for two different vertices, the vertex with the conduction velocity that has the smaller magnitude will be the vertex that has slower conduction velocity. Accordingly, in embodiments, the conduction velocity may satisfy the condition when the conduction velocity is no greater than a threshold. As another example, healthy cardiac activation may propagate at a speed of approximately 0.5 millimeters (mm) per millisecond (ms); and, unhealthy cardiac activation may propagate at roughly half the speed, e.g., approximately 0.25 mm/ms. As such, in embodiments where conduction velocity is used, a conduction velocity that is below 0.4 mm/ms and/or within 0.15 mm/ms of 0.25mm/ms may satisfy the condition. These are only examples, however, and not meant to be limiting.

Embodiments of method 500 also include causing the display of a cardiac map having representations of signal features corresponding to the set of conduction characteristics that satisfy the condition (block 514). The cardiac map that is displayed may be generated as described above in block 506. In embodiments, however, only signal features that correspond to the set of conduction characteristics that satisfy the condition may be displayed. As such, it may be easier for a physician and/or technician to identify especially relevant portions of the cardiac map (e.g., slow pathways), which may result in faster and more accurate treatment of a patient. In embodiments, the cardiac map may be displayed on a display device, for example, the display device 118 shown in FIG. 1 above.

FIG. 6 is a flow diagram depicting another illustrative method for facilitating display of cardiac mapping information, in accordance with embodiments of the present disclosure. Embodiments of method 600 may be performed, in whole or in part, by a mapping system (e.g., the mapping system 100 depicted in FIG. 1A). Embodiments of the method 600 include receiving, from a mapping probe, a number of cardiac electrical signals (block 602) and determining a number of signal features, each signal feature including a feature of one of the sensed cardiac signals (block 604). Each of the sensed cardiac electrical signals (and, accordingly, features of each signal) corresponds to a respective point (e.g., location in space) where the cardiac electrical signal was sensed.

The sensed cardiac electrical signals can include a number of intracardiac EGMs. Examples of features of the cardiac electrical signals include, but are not limited to, activation times, minimum voltage values, maximum voltage values, maximum negative time-derivatives of voltages, instantaneous potentials, voltage amplitudes, dominant frequencies, peak-to-peak voltages, and/or the like. Each of the respective points at which a cardiac electrical signal is sensed has a corresponding set of three-dimensional position coordinates. For example, the position coordinates of the points may be represented in Cartesian coordinates. Other coordinate systems can be used, as well. In embodiments, an arbitrary origin is used and the respective position coordinates are defined with respect to the arbitrary origin. In embodiments, the point corresponding to each sensed cardiac electrical signal may be located on the endocardial surface of the heart and/or below the endocardial surface of the heart.

Embodiments of method 600 include determining conduction characteristics using the activation times corresponding to electrical signals sensed by three electrodes (block 606). In embodiments, conduction characteristics may be activation gradients or conduction velocities. In embodiments, using a least-squares fit computation, the relative activation times and positions extracted from one or more cardiac signals can be used to determine the propagation vector for the activation signal. Similar to above, the direction of the propagation vector is the direction of propagation of the activation signal and the reciprocal of the magnitude of the propagation vector corresponds to the conduction velocity of the activation signal. In embodiments, the signal used to determine the activation time gradients and/or the conduction velocities may be from a signal cycle (or "beat"). Determining conduction characteristics based on activation times corresponding to cardiac electrical signals sensed by three electrodes may be referred to herein, interchangeably, as a "pre-filter" method for determining conduction characteristics.

In embodiments, determining conduction characteristics using the pre-filter method may have advantages. For example, in general, a cardiac map is generated from signals that are sensed from multiple beats. One or more of these signals may be used as one or more references and the other signals that are sensed may be aligned relative to the one or more reference signals. During this alignment process, noise may be introduced because the alignments of the signals may not exact due to slight aberrations in the signals sensed from different beats. Using the pre-filter method, however, this alignment noise is not introduced since the cardiac signal from which the activation times are extracted may be from a single beat.

Another advantage to the pre-filter method may be when generating a cardiac map. For example, when constructing an activation map, instead of only determining the most prevalent activation time for a specific point (or vertex) and using that activation time to generate an activation map, a direction, as determined using the pre-filtered conduction characteristic, may be associated with each activation time. As such, if two different activation times are extracted from cardiac signals over multiple beats at approximately the same frequency and position, eliminating one of the activation times may be easier to do based on the direction associated with the activation time and an expected activation propagation. For example, assume a first activation time of 10 ms (relative to a reference time) and a second activation time of 20 ms (relative to the reference time) are extracted from a cardiac signal at a vertex. Further assume that the first activation time has a direction of 45 degrees relative to a reference angle and the second activation time has a direction of 90 degrees relative to the reference angle. If activation signals surrounding the vertex have a direction of 45 relative to a reference angle, then the second activation time may be discarded because it has a direction that does not match the directions of activation signals surrounding the vertex. Accordingly, the pre-filter method may be used to improve certain cardiac maps by rejecting some extracted features that do not align with other extracted features.

Embodiments of method 600 include determining a set of conduction characteristics that satisfy a condition (block 608). In embodiments, the condition may be selected based on whether the conduction characteristic is a conduction velocity or an activation time gradient and what portion of a cardiac map a physician and/or technician would like to see. For example, as described above, reentry of an activation signal, which may result in an arrhythmia, may be due to a slow pathway. As such, the condition for the conduction characteristic may be set to distinguish between a propagation speed that is typical of healthy cardiac tissue and a propagation speed that is typical of unhealthy cardiac tissue. To distinguish between propagation speeds, a range of conduction characteristic values that is typical of unhealthy cardiac tissue may satisfy the condition and/or conduction characteristic values that are above or below a threshold value may satisfy the condition.

For example, when the conduction characteristic are activation time gradients and when comparing the activation time gradient for two different vertices, the vertex with the activation time gradient that has the larger magnitude will be the vertex that has slower conduction velocity. Accordingly, in embodiments, the activation gradient may satisfy the condition when the activation gradient is no less than a threshold. And, conversely, when the conduction characteristics are conduction velocities and when comparing the conduction velocities for two different vertices, the vertex with the conduction velocity that has the smaller magnitude will be the vertex that has slower conduction velocity. Accordingly, in embodiments, the conduction velocity may satisfy the condition when the conduction velocity is no greater than a threshold. As another example, healthy cardiac activation may propagate at a speed of approximately 0.5 millimeters (mm) per millisecond (ms); and, unhealthy cardiac activation may propagate at roughly half the speed, e.g., approximately 0.25 mm/ms. As such, in embodiments where the conduction characteristic is a conduction velocity, a conduction velocity that is below 0.4 mm/ms and/or within 0.15 mm/ms of 0.25mm/ms may satisfy the condition. These are only examples, however, and not meant to be limiting.

Embodiments of method 600 also include generating a cardiac map (block 610). In embodiments, the cardiac map may be generated based, at least in part, on the cardiac electrical signal features and may include an anatomical shell and a representation of at least one of the signal features. In embodiments, some signal features may be rejected based on the determined pre-filtered conduction characteristic, as described above in the discussion of block 606 and, therefore, not used to generate the cardiac map. In embodiments, the cardiac map may also be generated, at least in part, using any number of other signals, techniques, and/or the like. For example, embodiments may utilize impedance mapping techniques to generate one or more portions of the cardiac map such as, for example, an anatomical shell upon which electrical signal features are represented. In embodiments, a surface may be fitted on one or more of the points associated with the cardiac electrical signals to generate a shell representing the endocardial surface of the one or more cardiac structures. In embodiments, a surface may also be fitted on one or more of the points associated with the cardiac electrical signals to generate a shell representing an epicardium surface or other excitable cardiac tissue. In embodiments, the surface geometry of the shell can be represented as a mesh containing a collection of vertices (points) and the connectivity between them (e.g. triangles). Alternatively, surface geometry can be represented by different functions such as higher order meshes, non-uniform rational basis splines (NURBS), and/or curvilinear shapes.

In embodiments, one or more of the cardiac electrical signal features at the corresponding points can be included on the shell to generate a cardiac map of the one or more cardiac structures. For example, embodiments may include displaying annotations on the cardiac map that represent features, extracted from the cardiac electrical signals and/or derived from other features, such as, for example, activation times, minimum voltage values, maximum voltage values, maximum negative time-derivatives of voltages, instantaneous potentials, voltage amplitudes, dominant frequencies, peak-to-peak voltages, and/or the like.

Embodiments of method 600 also include causing the display of a cardiac map having representations of signal features corresponding to the set of conduction characteristics that satisfy the condition (block 614). The cardiac map that is displayed may be generated as described above in block 610. In embodiments, however, only signal features that correspond to the set of conduction characteristics that satisfy the condition may be displayed. As such, it may be easier for a physician and/or technician to identify especially relevant portions of the cardiac map (e.g., slow pathways), which may result in faster and more accurate treatment of a patient. In embodiments, the cardiac map may be displayed on a display device, for example, the display device 118 shown in FIG. 1 above.

Various modifications and additions can be made to the exemplary embodiments discussed without departing from the scope of the present disclosure. For example, while the embodiments described above refer to particular features, the scope of this disclosure also includes embodiments having different combinations of features and embodiments that do not include all of the described features. Accordingly, the scope of the present invention is intended to embrace all such alternatives, modifications, and variations as fall within the scope of the appended claims.

## Claims

1. A system (100) for facilitating display of cardiac mapping information, the system (100) comprising:
a mapping probe (106) configured to sense a plurality of cardiac electrical signals; and
a processing unit (110) configured to:
receive the plurality of cardiac electrical signals;
determine a plurality of signal features, each of the plurality of signal features comprising a feature of one of the plurality of cardiac electrical signals;
determine a plurality of conduction characteristics that satisfy a condition, each of the plurality of conduction characteristics based on one or more of the plurality of signal features, wherein the plurality of conduction characteristics are conduction velocities and wherein a conduction characteristic satisfies the condition when the conduction characteristic is no greater than a threshold; and
generate, based on the plurality of signal features and the corresponding plurality of conduction characteristics, a cardiac map, the cardiac map comprising an anatomical shell and a set of annotations representing at least a portion of the plurality of signal features, wherein the cardiac map does not include a representation of a signal feature that does not correspond to one of the set of conduction characteristics that satisfy the condition.

2. The system (100) of claim 1, wherein the processing unit (110) is further configured to determine a set of conduction characteristics, of the plurality of conduction characteristics, that satisfy a condition, wherein the set of annotations comprises a representation of each of the plurality of signal features that corresponds to one of the set of conduction characteristics that satisfy the condition.

3. The system (100) of any one of claims 1-2, wherein a conduction characteristic satisfies the condition when the conduction characteristic is within a particular range.

4. The system (100) of any one of claims 1-3, wherein each of the plurality of signal features comprises at least one of an activation time, a minimum voltage value, a maximum voltage value, a maximum negative time-derivative of voltage, an instantaneous potential, a voltage amplitude, a dominant frequency, a mean cycle length, and a peak-to-peak voltage.

5. The system (100) of claim 4, each of the plurality of signal features comprising an activation time, and wherein the processing unit (110) is configured to determine the plurality of conduction characteristics by:
determining a gradient associated with each of the plurality of activation times; and
determining the inverse of the gradient associated with each of the plurality of activation times.

6. The system (100) of claim 5, wherein each of the plurality of signal features comprises an activation time associated with a single beat.

7. The system (100) of claim 6, wherein the processing unit (110) is configured to determine the plurality of conduction characteristics by performing a least-squares computation using at least three electrode activation times, wherein each of the at least three electrode activation times is associated with one of at least three non-collinear electrode locations.

8. A method for facilitating display of cardiac mapping information, the method, which is performed by a processing unit, comprising:
receiving, from a mapping probe (106), a plurality of cardiac electrical signals;
determining a plurality of signal features, each of the plurality of signal features comprising a feature of one of the plurality of cardiac electrical signals;
determining a plurality of conduction characteristics that satisfy a condition, each of the plurality of conduction characteristics based on one or more of the plurality of signal features, wherein the plurality of conduction characteristics are conduction velocities and wherein a conduction characteristic satisfies the condition when the conduction characteristic is no greater than a threshold; and
causing a display device to display, based on the plurality of conduction characteristics, a cardiac map, the displayed cardiac map comprising an anatomical shell and a representation of at least one of the plurality of signal features, wherein the cardiac map does not include a representation of a signal feature that does not correspond to one of the set of conduction characteristics that satisfy the condition.

9. The method of claim 8, further comprising:
generating an activation map, the activation map comprising the anatomical shell, a plurality of vertices and a representation of each of a plurality of activation times at a vertex of the plurality of vertices; and
determining activation time differences for each vertex, wherein each of the plurality of conduction characteristics is determined based on one or more of the activation time differences.

10. The method of claim 9, wherein determining an activation time difference of a vertex includes performing a least-squares computation using at least three activation times associated with the vertex and at least two other map vertices of the activation map.

11. The method of claim 8, wherein determining the plurality of conduction characteristics includes: performing a least-squares computation using at least three electrode activation times, wherein each of the at least three electrode activation times is associated with one of at least three non-collinear electrode locations of the mapping probe (106).

## Patentansprüche

1. System (100), das die Darstellung von Kardial-Mapping-Informationen ermöglicht, wobei das System (100) umfasst:
eine Mapping-Sonde (106), die dafür ausgelegt ist, mehrere kardiale elektrische Signale zu erfassen; und
eine Verarbeitungseinheit (110), die für folgendes ausgelegt ist:
Empfangen der mehreren kardialen elektrischen Signale;
Bestimmen mehrerer Signalmerkmale, wobei jedes von den mehreren Signalmerkmalen ein Merkmal von jeweils einem von den mehreren kardialen elektrischen Signalen umfasst;
Bestimmen mehrerer Leiteigenschaften, die eine Bedingung erfüllen, wobei jede von den mehreren Leiteigenschaften auf einem oder mehreren von den mehreren Signalmerkmalen basiert, wobei die mehreren Leiteigenschaften Leitgeschwindigkeiten sind, und wobei eine Leiteigenschaft die Bedingung erfüllt, wenn die Leiteigenschaft nicht größer ist als ein Schwellenwert; und
Erzeugen einer kardialen Abbildung auf Basis der mehreren Signalmerkmale und der entsprechenden mehreren Leiteigenschaften, wobei die kardiale Abbildung eine anatomische Hülle und einen Satz von Annotationen umfasst, die zumindest einen Teil der mehreren Signalmerkmale darstellen, wobei die kardiale Abbildung keine Darstellung eines Signalmerkmals enthält, das keiner von dem Satz von Leiteigenschaften entspricht, welche die Bedingung erfüllen.

2. System (100) nach Anspruch 1, wobei die Verarbeitungseinheit (110) ferner dafür ausgelegt ist, unter den mehreren Leiteigenschaften einen Satz von Leiteigenschaften, die eine Bedingung erfüllen, zu bestimmen, wobei der Satz von Annotationen eine Darstellung von jedem von den mehreren Signalmerkmalen umfasst, das einer von dem Satz von Leiteigenschaften, welche die Bedingung erfüllen, entspricht.

3. System (100) nach einem der Ansprüche 1-2, wobei eine Leiteigenschaft die Bedingung erfüllt, wenn die Leiteigenschaft in einem bestimmten Bereich liegt.

4. System (100) nach einem der Ansprüche 1-3, wobei jedes von den mehreren Signalmerkmalen mindestens eines der folgenden umfasst: eine Aktivierungszeit, ein kleinster Spannungswert, ein größter Spannungswert, eine größte negative Zeitableitung der Spannung, ein momentanes Potential, eine Spannungsamplitude, eine dominante Frequenz, eine mittlere Zykluslänge und eine Spitzenspannung.

5. System (100) nach Anspruch 4, wobei jedes von den mehreren Signalmerkmalen eine Aktivierungszeit umfasst, und wobei die Verarbeitungseinheit (110) dafür ausgelegt ist, die mehreren Leiteigenschaften zu bestimmen durch:
Bestimmen eines Gradienten, der mit jeder von den mehreren Aktivierungszeiten assoziiert ist; und
Bestimmen des Kehrwerts des Gradienten, der mit jeder von den mehreren Aktivierungszeiten assoziiert ist.

6. System (100) nach Anspruch 5, wobei jedes von den mehreren Signalmerkmalen eine Aktivierungszeit umfasst, die mit einem einzelnen Herzschlag assoziiert ist.

7. System (100) nach Anspruch 6, wobei die Verarbeitungseinheit (110) dafür ausgelegt ist, die mehreren Leiteigenschaften durch Durchführen einer Berechnung von kleinsten Quadraten unter Verwendung von mindestens drei Elektrodenaktivierungszeiten zu bestimmen, wobei jede von den mindestens drei Elektrodenaktivierungszeiten mit jeweils einem von mindestens drei nicht-kollinearen Elektrodenorten assoziiert ist.

8. Verfahren, um ein Anzeigen von Kardial-Mapping-Informationen zu ermöglichen, wobei das Verfahren, das durch eine Verarbeitungseinheit durchgeführt wird, umfasst:
Empfangen mehrerer kardialer elektrischer Signale von einer Mapping-Sonde (106);
Bestimmen mehrerer Signalmerkmale, wobei jedes von den mehreren Signalmerkmalen ein Merkmal von einem von den mehreren kardialen elektrischen Signalen umfasst;
Bestimmen mehrerer Leiteigenschaften, die eine Bedingung erfüllen, wobei jede von den mehreren Leiteigenschaften auf einem oder mehreren von den mehreren Signalmerkmalen basiert, wobei die mehreren Leiteigenschaften Leitgeschwindigkeiten sind und wobei eine Leiteigenschaft die Bedingung erfüllt, wenn die Leiteigenschaft nicht größer ist als ein Schwellenwert; und
Bewirken, dass eine Anzeigevorrichtung auf Basis der mehreren Leiteigenschaften eine kardiale Abbildung anzeigt, wobei die angezeigte kardiale Abbildung eine anatomische Hülle und eine Darstellung von mindestens einem von den mehreren Signalmerkmalen umfasst, wobei die kardiale Abbildung keine Darstellung eines Signalmerkmals enthält, das keiner von dem Satz von Leiteigenschaften entspricht, welche die Bedingung erfüllen.

9. Verfahren nach Anspruch 8, ferner umfassend:
Erzeugen einer Aktivierungsabbildung, wobei die Aktivierungsabbildung die anatomische Hülle umfasst, mehrerer Scheitelpunkte und einer Darstellung von jeder von den mehreren Aktivierungszeiten an einem Scheitelpunkt von den mehreren Scheitelpunkten; und
Bestimmen von Aktivierungszeitdifferenzen für jeden Scheitelpunkt, wobei jede von den mehreren Leiteigenschaften auf Basis einer oder mehrerer von den Aktivierungszeitdifferenzen bestimmt wird.

10. Verfahren nach Anspruch 9, wobei das Bestimmen einer Aktivierungszeitdifferenz eines Scheitelpunkts das Durchführen einer Berechnung kleinster Quadrate unter Verwendung von mindestens drei Aktivierungszeiten, die mit dem Scheitelpunkt assoziiert sind, und von mindestens zwei anderen Abbildungsscheitelpunkten der Aktivierungsabbildung umfasst.

11. Verfahren nach Anspruch 8, wobei das Bestimmen der mehreren Leiteigenschaften beinhaltet: Durchführen einer Berechnung von kleinsten Quadraten unter Verwendung von mindestens drei Elektrodenaktivierungszeiten, wobei jede von den mindestens drei Elektrodenaktivierungszeiten mit jeweils einem von mindestens drei nicht-kollinearen Elektrodenorten der Mapping-Sonde (106) assoziiert ist.

## Revendications

1. Système (100) pour faciliter l'affichage d'une information de cartographie cardiaque, le système (100) comprenant :
une sonde de cartographie (106) qui est configurée pour détecter une pluralité de signaux électriques cardiaques ; et
une unité de traitement (110) qui est configurée pour :
recevoir la pluralité de signaux électriques cardiaques ;
déterminer une pluralité de caractérisations de signal, chacune de la pluralité de caractérisations de signal comprenant une caractérisation de l'un de la pluralité de signaux électriques cardiaques ;
déterminer une pluralité de caractéristiques de conduction qui satisfont une condition, chacune de la pluralité de caractéristiques de conduction étant basée sur une ou plusieurs caractérisation(s) de la pluralité de caractérisations de signal, dans lequel les caractéristiques de conduction de la pluralité de caractéristiques de conduction sont des vitesses de conduction et dans lequel une caractéristique de conduction satisfait la condition lorsque la caractéristique de conduction n'est pas supérieure à un seuil ; et
générer, sur la base de la pluralité de caractérisations de signal et de la pluralité correspondante de caractéristiques de conduction, une carte cardiaque, la carte cardiaque comprenant une enveloppe anatomique et un jeu d'annotations qui représentent au moins une partie de la pluralité de caractérisations de signal, dans lequel la carte cardiaque n'inclut pas une représentation d'une caractérisation de signal qui ne correspond pas à une caractéristique de conduction du jeu de caractéristiques de conduction qui satisfont la condition.

2. Système (100) selon la revendication 1, dans lequel l'unité de traitement (110) est en outre configurée pour déterminer un jeu de caractéristiques de conduction, parmi la pluralité de caractéristiques de conduction, qui satisfont une condition, dans lequel le jeu d'annotations comprend une représentation de chacune de la pluralité de caractérisations de signal qui correspond à une caractéristique de conduction du jeu de caractéristiques de conduction qui satisfont la condition.

3. Système (100) selon l'une quelconque des revendications 1 et 2, dans lequel une caractéristique de conduction satisfait la condition lorsque la caractéristique de conduction est à l'intérieur d'une plage particulière.

4. Système (100) selon l'une quelconque des revendications 1 à 3, dans lequel chacune de la pluralité de caractérisations de signal comprend au moins un paramètre pris parmi un temps d'activation, une valeur de tension minimum, une valeur de tension maximum, une dérivée de la tension par rapport au temps négative maximum, un potentiel instantané, une amplitude de tension, une fréquence dominante, une longueur de cycle moyenne et une tension crête à crête.

5. Système (100) selon la revendication 4, dans lequel chacune de la pluralité de caractérisations de signal comprend un temps d'activation, et dans lequel l'unité de traitement (110) est configurée pour déterminer la pluralité de caractéristiques de conduction en réalisant les actions qui suivent :
la détermination d'un gradient qui est associé à chacun de la pluralité de temps d'activation ; et
la détermination de l'inverse du gradient qui est associé à chacun de la pluralité de temps d'activation.

6. Système (100) selon la revendication 5, dans lequel chacune de la pluralité de caractérisations de signal comprend un temps d'activation qui est associé à un unique battement.

7. Système (100) selon la revendication 6, dans lequel l'unité de traitement (110) est configurée pour déterminer la pluralité de caractéristiques de conduction en réalisant un calcul par les moindres carrés qui utilise au moins trois temps d'activation d'électrode, dans lequel chacun des au moins trois temps d'activation d'électrode est associé à une localisation d'au moins trois localisations d'électrode non colinéaires.

8. Procédé pour faciliter l'affichage d'une information de cartographie cardiaque, lequel procédé est réalisé par une unité de traitement, comprenant :
la réception, en provenance d'une sonde de cartographie (106), d'une pluralité de signaux électriques cardiaques ;
la détermination d'une pluralité de caractérisations de signal, chacune de la pluralité de caractérisations de signal comprenant une caractérisation de l'un de la pluralité de signaux électriques cardiaques ;
la détermination d'une pluralité de caractéristiques de conduction qui satisfont une condition, chacune de la pluralité de caractéristiques de conduction étant basée sur une ou plusieurs caractérisation(s) de la pluralité de caractérisations de signal, dans lequel les caractéristiques de conduction de la pluralité de caractéristiques de conduction sont des vitesses de conduction et dans lequel une caractéristique de conduction satisfait la condition lorsque la caractéristique de conduction n'est pas supérieure à un seuil ; et
le fait de forcer un dispositif d'affichage à afficher, sur la base de la pluralité de caractéristiques de conduction, une carte cardiaque, la carte cardiaque affichée comprenant une enveloppe anatomique et une représentation d'au moins l'une de la pluralité de caractérisations de signal, dans lequel la carte cardiaque n'inclut pas une représentation d'une caractérisation de signal qui ne correspond pas à une caractéristique de conduction du jeu de caractéristiques de conduction qui satisfont la condition.

9. Procédé selon la revendication 8, comprenant en outre :
la génération d'une carte d'activation, la carte d'activation comprenant l'enveloppe anatomique, une pluralité de vertex et une représentation de chacun d'une pluralité de temps d'activation au niveau d'un vertex de la pluralité de vertex ; et
la détermination de différences de temps d'activation pour chaque vertex, dans lequel chacune de la pluralité de caractéristiques de conduction est déterminée sur la base d'une ou de plusieurs différence(s) de temps d'activation des différences de temps d'activation.

10. Procédé selon la revendication 9, dans lequel la détermination d'une différence de temps d'activation d'un vertex inclut la réalisation d'un calcul par les moindres carrés qui utilise au moins trois temps d'activation d'électrode qui sont associés au vertex et à au moins deux autres vertex de carte de la carte d'activation.

11. Procédé selon la revendication 8, dans lequel la détermination de la pluralité de caractéristiques de conduction inclut : la réalisation d'un calcul par les moindres carrés qui utilise au moins trois temps d'activation d'électrode, dans lequel chacun des au moins trois temps d'activation d'électrode est associé à une localisation d'au moins trois localisations d'électrode non colinéaires de la sonde de cartographie (106).
